(19) Europäisches Patentamt European Patent Office Office européen des brevets

(11) **EP 2 022 859 A1**

(12) **EUROPÄISCHE PATENTANMELDUNG**

(43) Veröffentlichungstag:
**11.02.2009 Patentblatt 2009/07**

(51) Int Cl.:
*C12Q 1/32* *(2006.01)*     *C12Q 1/54* *(2006.01)*

(21) Anmeldenummer: 07015084.2

(22) Anmeldetag: **01.08.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(71) Anmelder:
• **Roche Diagnostics GmbH**
 **68305 Mannheim (DE)**
 Benannte Vertragsstaaten:
 **DE**
• **F.HOFFMANN-LA ROCHE AG**
 **4070 Basel (CH)**
 Benannte Vertragsstaaten:
 **AT BE BG CH CY CZ DK EE ES FI FR GB GR HU IE IS IT LI LT LU LV MC MT NL PL PT RO SE SI SK TR**

(72) Erfinder:
• **Petrich, Wolfgang**
 **76669 Bad Schönborn (DE)**
• **Haar, Hans-Peter**
 **69168 Wiesloch (DE)**
• **Hoenes, Joachim**
 **64673 Zwingenberg (DE)**
• **Horn, Carina**
 **68647 Biblis (DE)**

(74) Vertreter: **Pfeifer, Hans-Peter et al**
 **Durm & Partner**
 **Patentanwälte**
 **Moltkestrasse 45**
 **76133 Karlsruhe (DE)**

(54) **Verfahren und Vorrichtung zur Bestimmung der Konzentration eines Analyten mittels Fluoreszenzmessung**

(57) Verfahren zur Bestimmung der Konzentration eines Analyten in einer flüssigen Probe mittels eines Reagenzsystems, wobei die Reaktion der Probe mit dem Reagenzsystem zu einer Änderung der Menge eines Fluorophors führt, die Menge des Fluorophors mit der Konzentration des Analyten korreliert, eine für die Menge des Fluorophors charakteristische Messgröße gemessen und die Konzentration des Analyten mittels eines Auswertealgorithmus auf Basis der Messgröße ermittelt wird. Das Verfahren zeichnet sich dadurch aus, dass die Fluoreszenzlebensdauer des Fluorophors bei der Ermittlung der Konzentration des Analyten verwendet wird.

EP 2 022 859 A1

**Beschreibung**

Die Erfindung betrifft ein Verfahren zur Bestimmung der Konzentration eines Analyten in einer flüssigen Probe. Insbesondere geht es um die Untersuchung medizinischer Proben, vor allem von Blut und/oder interstitieller Flüssigkeit auf darin enthaltene Analyten. Ein besonders wichtiger Analyt ist Glucose.

Speziell richtet sich die Erfindung auf Verfahren, bei denen die Ermittlung der Konzentration des Analyten auf einer Fluoreszenzmessung basiert. Bei solchen Verfahren wird ein Reagenzsystem verwendet, dessen Reaktion mit der Probe zu einer Änderung der Menge eines Fluorophors führt, wobei die Mengenänderung grundsätzlich sowohl eine Zunahme durch Bildung eines Fluorophors, als auch eine Abnahme durch Verbrauch eines Fluorophors sein kann. Das Reaktionssystem ist dabei so gewählt, dass die Änderung der Menge des Fluorophors für die gewünschte analytische Konzentration charakteristisch ist. Das Analyseverfahren schließt die Messung einer mit der Menge des Fluorophors korrelierenden Messgröße ein. Auf Basis der resultierenden Messwerte wird mittels eines Auswertealgorithmus die Konzentration ermittelt.

Derartige Verfahren sind bekannt, wobei beispielsweise auf folgende Dokumente verwiesen werden kann:

(1) EP 0 293 732 A2
(2) US 2005/0214891 A1
(3) US 2006/0003397 A1
Alle diese Publikationen beschreiben ein ähnliches Testprotokoll, bei dem das Reagenzsystem das Enzym-Coenzym-Paar Glucosedehydrogenase (GlucDH) / Nicotinamid-Adenin-Dinucleotid (NAD) enthält. Bei der Reaktion mit diesem Reagenzsystem wird unter dem Einfluss der GlucDH ein Hydridion aus der Glucose abgespalten und auf das NAD übertragen, so dass sich NADH bildet. Die gebildete Menge NADH ist charakteristisch für die Glucosekonzentration. NADH ist ein starker Fluorophor, dessen Konzentration durch eine Messung der Fluoreszenzintensität bestimmt werden kann. Die Korrelation der gemessenen Fluoreszenzintensität mit der Analytkonzentration wird üblicherweise durch Kalibration bestimmt. NADH ist auch ein Farbstoff. Deshalb kann es sinnvoll sein, ergänzend eine photometrische Messung (beispielsweise Messung der optischen Absorption bei einer bestimmten Wellenlänge) durchzuführen.

Diese Verfahrensweise ist grundsätzlich schon seit langem bekannt. Beispielsweise wird in dem Dokument (2) auf eine Publikation von Narayanaswamy et al. aus dem Jahr 1988 verwiesen, in der eine Fluoreszenzmessung mit GlucDH und NAD zur Glucosebestimmung verwendet worden sei. Mit der Messung der Fluoreszenzintensität ist jedoch eine Reihe von grundlegenden Problemen verbunden, die beispielsweise in dem Buch
(4) J.R. Lakovicz "Principles of Fluorescence Spectroscopy" Springer Science and Business Media, 2006

diskutiert werden:

- Die Intensität des detektierten Fluoreszenzsignals hängt von mehreren instrumentenspezifischen Faktoren ab, wie beispielsweise der Leistung der Lichtquelle, der Transmission der optischen Komponenten im Lichtweg oder der Sensitivität des Detektors. Unkontrollierte Änderungen dieser Faktoren beeinträchtigen die Messgenauigkeit.

- Eine weitere Fehlerquelle bei Intensitätsmessungen resultiert aus nicht spezifischem Licht, das aus der Umgebung an den Detektor dringt und eine unkontrollierte Signaländerung bewirken kann.

- Die Intensität des gemessenen Fluoreszenzlichts ist nicht nur von der Menge des Fluorophors abhängig, sondern wird auch von dessen molekularer Umgebung in der Probe erheblich beeinflusst. Dazu tragen insbesondere Prozesse bei, die unter dem Begriff Fluoreszenzlöschung ("quenching") zusammengefasst werden.

- Da zwischen der Anregung eines Moleküls und der Abstrahlung eines Lichtquants im statistischen Mittel eine Zeit in der Größenordnung von Nanosekunden vergeht, kann sich die Position und Orientierung des Moleküls zwischen Absorption und Emission ändern.
Daraus resultieren Störeinflüsse hinsichtlich der Fluoreszenzintensität, insbesondere eine Temperaturabhängigkeit.

- Häufig erfolgt die Anregung der Fluoreszenz durch ultraviolettes Licht. Dabei können photochemische Reaktionen des elektronisch angeregten Zustandes zu einem Ausbleichen des Fluorophors ("bleaching") führen. Dies ist eine weitere Fehlerquelle.

Eine Verminderung dieser Probleme lässt sich dadurch erreichen, dass eine Kalibration der Messung der Fluoreszenzintensität mit Hilfe eines Fluorophors, dessen Fluoreszenzintensität bekannt ist, durchgeführt wird. Dies ist jedoch sehr aufwendig und eignet sich nur für anspruchsvolle Labormessungen.

Auf dieser Grundlage liegt der Erfindung das technische Problem zugrunde, ein Verfahren vorzuschlagen, das eine verbesserte Messgenauigkeit, insbesondere hinsichtlich der erwähnten Messfehler und Störungen mit vermindertem Aufwand ermöglicht. Es soll sich insbesondere auch für Kleingeräte eignen, die möglichst einfach und kostengünstig arbeiten.

Dieses technische Problem wird gelöst durch ein Verfahren der eingangs erläuterten Art, das dadurch gekennzeichnet ist, dass die Fluoreszenzlebensdauer des Fluorophors bei der Ermittlung der Konzentration des Analyten verwendet wird. Gegenstand der Erfindung ist auch ein Analysesystem zur Durchführung des Verfahrens.

Im Rahmen der Erfindung wurde festgestellt, dass die Fluoreszenzlebensdauer überraschenderweise in einem so starken Ausmaß von der Analytkonzentration abhängt, dass Messwerte dieser Messgröße vorteilhaft in dem Algorithmus zur Ermittlung des analytischen Ergebnisses (gesuchte Konzentration) verwendet werden können. In Kenntnis der Erfindung lässt sich dies für den Fall eines Glucosetests mit dem Reagenzsystem GlucDH/NAD folgendermaßen erklären.

Das infolge der Reaktion der Glucose mit dem Enzym-Coenzym-Paar GlucDH/NAD gebildete NADH bildet mit der GlucDH einen Komplex. Diese Komplexbildung beeinflusst die Fluoreszenzlebensdauer des NADH: Die Fluoreszenz des freien NADH ist kurzlebig, weil Moleküle in der umgebenden Flüssigkeit als Quencher wirken. In dem Komplex ist das NADH-Molekül gegen die Wirkung der Quencher weitgehend geschützt und die Fluoreszenz erheblich langlebiger.

Diese Tatsachen sind aus der Literatur bekannt. Völlig unerwartet war hingegen, dass diese Effekt unter den in praktischen Tests vorherrschenden Bedingung so ausgeprägt ist, dass die in der Praxis auftretenden Änderungen der Analytkonzentration in Blutproben zu einer gut messbaren Verschiebung der mittleren Fluoreszenzlebensdauer führen. Aufgrund der Literaturangaben, insbesondere in dem Dokument (3), wäre zu erwarten gewesen, dass der Bindungsgrad des Komplexes NADH/GlucDH sehr hoch ist. Das von dem Komplex NADH/GlucDH emittierte Fluoreszenzlicht hat eine sehr viel größere Intensität als die Fluoreszenz von freier NADH. Deswegen wäre zu erwarten gewesen, dass die beobachtete Fluoreszenzlebensdauer nur in sehr geringem Ausmaß von der freien NADH abhängig und der mit Konzentrationsänderungen im physiologischen Bereich verbundene Effekt hinsichtlich der Lebensdauer unmessbar gering ist. Im Rahmen der Erfindung wurde jedoch festgestellt, dass unter Praxisbedingungen ein sehr hoher Anteil des NADH (über 90%) unkomplexiert vorliegt.

Die Fluoreszenzlebensdauer liefert zusätzlich zu der Fluoreszenz-intensität eine zweite, unabhängige Information über die Fluoreszenz der Reaktionsprodukte. Sie kann selbständig (also ohne andere Informationen) zur Ermittlung der gesuchten Analytkonzentration verwendet werden. Vorzugsweise wird sie jedoch in Kombination mit der Messung der Fluoreszenzintensität eingesetzt. Dies erfordert keinen zusätzlichen messtechnischen Aufwand, weil übliche Verfahren zur Messung der Fluoreszenzlebensdauer zugleich Messergebnisse über die Fluoreszenzintensität liefern.

Um die Fluoreszenzlebensdauer erfindungsgemäß bei der Bestimmung der gesuchten Analytkonzentration zu verwenden, kann es zweckmäßig sein, sie zu messen und die aus der Messung resultierenden Messwerte in geeigneter Weise innerhalb des Auswertealgorithmus zu berücksichtigen. Eine solche Messung (im strengen Sinne, d. h. Messung von zuvor unbekannten Messwerten) ist jedoch nicht notwendig. Vielmehr kann die Fluoreszenzlebensdauer auch in einer Weise verwendet werden, bei der keine Messung in dem Sinn notwendig ist, dass (zuvor unbekannte) Messwerte der Fluoreszenzlebensdauer bestimmt werden. Eine Möglichkeit besteht darin, die Fluoreszenzintensität innerhalb eines definierten Zeitfensters zu messen, wobei das Zeitfenster an die (bekannte) Fluoreszenzlebensdauer eines aus der Reaktion der Probe mit dem Reagenzsystem resultierenden Fluorophors angepasst ist. Der aus einer solchen Messung resultierende Messwert steht in Beziehung zu der Fluoreszenzlebensdauer und wird nachfolgend auch als Lebensdauerbezogene Fluoreszenzintensität bezeichnet. Da er in dem Auswertealgorithmus verwendet wird, kann auch in diesem Fall davon gesprochen werden, dass die Fluoreszenzlebensdauer in dem Auswertealgorithmus verwendet wird.

Da demzufolge eine Messung der Fluoreszenzlebensdauer (in dem erläuterten strengen Sinn) nicht unbedingt notwendig ist, muss das Auswertegerät eines entsprechenden Analysesystems auch nicht notwendigerweise eine Messeinheit aufweisen, die dazu ausgebildet ist, die Fluoreszenzlebensdauer des Fluorophors zu messen. Vielmehr besteht alternativ oder zusätzlich die Möglichkeit, das die Messeinheit des Auswertegeräts so ausgebildet ist, dass sie die Fluoreszenzlebensdauer in anderer Weise, beispielsweise mittels des erwähnten bei der Intensitätsmessung gesetzten Zeitfensters, berücksichtigt.

Geeignete Verfahren zur Messung der Fluoreszenzlebensdauer sind bekannt. Grundsätzlich für die Erfindung geeignet ist eine zeitaufgelöste Fluoreszenzdetektion, bei der ein sehr kurzer Puls des Anregungslichts eingestrahlt und der Verlauf der resultierenden Emissionskurve mit geeigneten zeitlich hochauflösenden Detektionsverfahren detektiert wird. Vorzugsweise wird im Rahmen der Erfindung jedoch ein mit kontinuierlicher Einstrahlung arbeitendes Phasenmodulationsverfahren verwendet. Nähere Informationen über geeignete Verfahren können der Literatur entnommen werden. Zu verweisen ist insbesondere auf das als Dokument (4) zitierte Buch von Lakovicz sowie folgende weitere Publikationen und die darin zitierte Literatur:

(5) T. G. Scott et al. "Emission Properties of NADH. Studies of Fluorescence Lifetimes ..."; J. Am. Chem. Soc., 1970, 687 - 695

(6) US-patent 5,485,530

(7) EP 0 561 653 A1

Durch die Erfindung werden u.a. folgende Vorteile erreicht:

- Die oben angesprochenen Probleme werden weitgehend vermieden. Insbesondere ist keine Kalibration der Fluoreszenzintensität mittels eines Standard-Fluorophors notwendig.

- Die hochfrequente Messung führt zu einer Reduktion des Einflusses von störendem Umlicht.

- Die Erfindung ermöglicht eine erhöhte Genauigkeit, insbesondere durch Elimination oder Reduzierung von Fehlerquellen.

Die Erfindung wird nachfolgend anhand der Figuren näher erläutert. Die dargestellten und beschriebenen Besonderheiten der Erfindung können einzeln oder in Kombination verwendet werden, um bevorzugte Ausführungsformen der Erfindung zu schaffen. Es zeigen:

Fig. 1    eine Prinzipskizze der wesentlichen Funktionskomponenten eines für die Erfindung geeigneten Analysesystems;

Fig. 2    Messergebnisse hinsichtlich der mittleren Lebensdauer der Fluoreszenz in Abhängigkeit von dem Konzentrationsverhältnis zwischen GlucDH und NADH;

Fig.3    Messergebnis hinsichtlich des funktionalen Zusammenhangs zwischen der Glucosekonzentration und dem Intensitätsverhältnis zwischen kurzlebiger und langlebiger Fluoreszenz;

Fig. 4    einen Vergleich der erfindungsgemäß gemessenen Glucosekonzentration mit den Ergebnissen eines Referenzmessverfahrens.

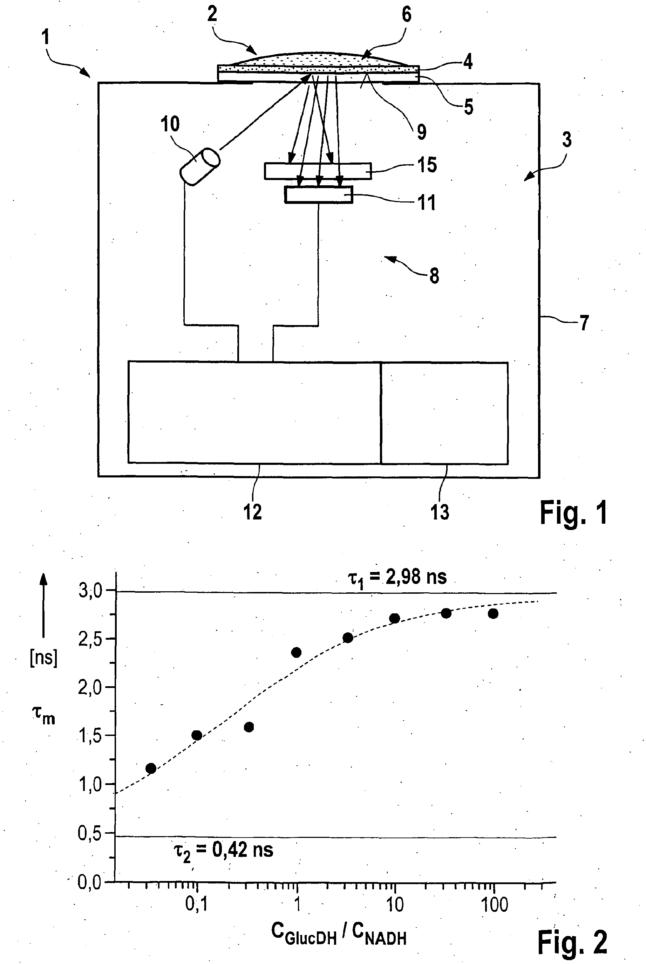

Das in Figur 1 stark schematisiert dargestellte Analysesystem besteht aus zwei aufeinander abgestimmten Komponenten, nämlich einem Analyseelement 2 und einem Auswertegerät 3. Das Analyseelement 2 umfasst eine Reagenzschicht 4, die ein Reagenzsystem in einer geeigneten Matrix, beispielsweise einer Gelmatrix, enthält. Die Reagenzschicht 4 befindet sich auf einem optisch transparenten Träger 5 Die

Reagenzien des Reagenzsystems sind in die Matrix der Reagenzschicht 4 in trockener Form eingebettet. Wenn eine Probenflüssigkeit 6 auf die freie Oberfläche der Reagenzschicht 4 aufgegeben wird und in diese eindringt, werden die Reagenzien des Reagenzsystems gelöst und es findet eine Reaktion der Probenflüssigkeit statt, die zu einer Änderung der Menge eines Fluorophors führt, welcher ein Bestandteil des Reagenzsystems ist. Der Aufbau solcher Analyseelemente wird beispielsweise in den Dokumenten US 3,802,842; US 4,061,468; US 2006/0003397 beschrieben.

Das Gehäuse 7 des Auswertegerätes 3 enthält eine insgesamt mit 8 bezeichnete Messeinheit, die geeignet ist, eine für die Menge des Fluorophors charakteristische Messgröße zu bestimmen. Die Messeinheit 8 schließt eine Anregungslichtquelle 10 ein, beispielsweise einen Laser oder eine LED, deren Licht von hinten (durch den optisch transparenten Träger 5) auf die von der Probenaufgabeseite abgewandte, dem optisch transparenten Träger 5 zugewandte Oberfläche der Reagenzschicht 4 gerichtet ist. Dabei wird von der Auswertezone 9 Sekundärlicht in Richtung auf einen Detektor 11 abgestrahlt und von diesem detektiert. Das resultierende Messsignal wird einer Signalverarbeitungseinheit zugeführt und in üblicher Weise verstärkt, aufbereitet und digitalisiert. Die resultierenden digitalisierten Messwerte werden einer Auswerteeinheit 13 zugeführt, die die erforderliche Soft- und Hardware enthält, um aus den digitalisierten Messwerten die gesuchte Konzentration des Analyten zu ermitteln.

Die Messeinheit 8 ist geeignet, von der Auswertezone 9 abgestrahltes Fluoreszenzlicht zu detektieren. Zu diesem Zweck wird mit üblichen Mitteln, beispielsweise einem in Figur 1 dargestellten Filterelement 15 der Zutritt des von der Anregungslichtquelle 10 ausgehenden Primärlichtes derartig blockiert, dass das mit einer höheren Wellenlänge abgestrahlte Fluoreszenzlicht selektiv von dem Detektor 11 erfasst werden kann.

Insoweit ist das erfindungsgemäße Analysesystem konventionell aufgebaut und muss deshalb nicht näher erläutert werden. Nähere Informationen können beispielsweise den zitierten Dokumenten des Standes der Technik entnommen werden.

Eine Besonderheit des in Figur 1 dargestellten erfindungsgemäßen Analysesystems besteht darin, dass die Messeinheit 8 dazu geeignet ist, die Fluoreszenzlebensdauer zu messen und/oder (im Rahmen der Messung einer anderen Messgröße) zu berücksichtigen. Wie bereits erwähnt, erfolgt die Fluoreszenzlebensdauer-Messung vorzugsweise mit einem Phasenmodulationsverfahren. Dabei wird das von der Anregungslichtquelle 10 abgestrahlte Licht hochfrequent moduliert und die gesuchte Information über die Fluoreszenzlebensdauer aus der mittels des Detektors 11 und der Signalverarbeitungseinheit 12 gemessenen Phasenverschiebung ermittelt. Auch diesbezüglich ist eine nähere Erläuterung nicht erforderlich, weil ergänzende Informationen der Literatur, insbesondere den oben erwähnten Dokumenten, entnommen werden können.

Figur 2 zeigt experimentelle Ergebnisse aus der der Erfindung zugrundeliegenden Forschungsarbeit. Dargestellt ist die mittlere Fluoreszenzlebensdauer $\tau_m$ für verschiedene Konzentrationsverhältnisse einer flüssigen Mischung von GlucDH und NADH. Einer NADH-Lösung mit einer Konzentration von 1,2 mg/l wurden variierende Mengen von GlucDH zugemischt, um die auf der Abszisse angegebenen Konzentrationen einzustellen. Figur 2 zeigt, dass die mit der Variation der Konzentration von GlucDH verbundene Verschiebung des Verhältnisses zwischen freier und komplexierter NADH durch Lebensdauermessungen gut beobachtet werden kann, wobei sich die Messkurve für sehr hohe und sehr niedrige GlucDH-Konzentrationen den Extremwerten $\tau_1$ = 2,98 ns und $\tau_2$ = 0,42 ns nähert.

Aus den in Figur 2 dargestellten Messergebnissen wurde im Rahmen der Erfindung abgeleitet, dass die Änderung der Lebensdauer als Information zur Quantifizierung eines Analyten in Fluoreszenztests verwendet werden kann. Diese Erkenntnis kann auf unterschiedliche Weise analytisch genutzt werden.

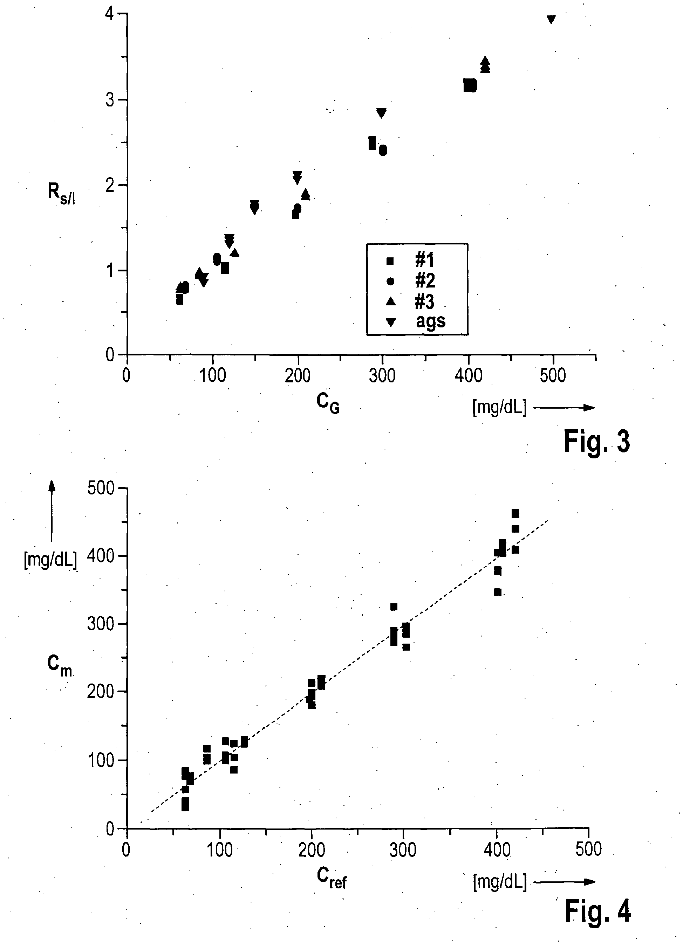

Figur 3 verdeutlicht eine bevorzugte Möglichkeit, bei der die Messung der Fluoreszenzlebensdauer dazu verwendet wird, eine Lebensdauer-bezogene Fluoreszenzintensität (Lifetime Related Fluorescence Intensity; LRFI) zu bestimmen. Ein solcher LRFI-Wert beschreibt die Intensität der Fluoreszenz in Abhängigkeit von der Fluoreszenzlebensdauer und bildet somit ein Maß für die Mengenrelation von fluoreszierenden Spezies, die sich durch unterschiedliche mittlere Lebensdauern auszeichnen, wie beispielsweise komplexiertes NADH im Verhältnis zu freiem NADH. Auf der Basis von durch kurze Lichtimpulse angeregten zeitlich hochaufgelöst gemessenen Fluoreszenzsignalen kann man einen LRFI-Wert beispielsweise dadurch ermitteln, dass der Abfall ("decay") des Fluoreszenzsignals eine logarithmische Funktion ist, die von den mittleren Fluoreszenzlebensdauern der fluoreszierenden Spezies und deren Konzentration abhängt. Die getrennten Intensitäten der beiden Signalkomponenten können durch mathematisches Fitten solcher Messkurven ermittelt werden.

Figur 3 zeigt die Abhängigkeit solcher LRFI-Werte von der Glucosekonzentration für einen Glucose-Fluoreszenztest mit Analyseelementen, wie sie in den oben genannten Dokumenten (2) und (3) beschrieben sind. Dargestellt ist ein Verhältnis $R_{s/l}$ zwischen dem LRFI-Wert für kurzlebiges freies NADH und dem LRFI-Wert für langlebiges komplexiertes NADH. Die Messungen wurden mit Blutproben unterschiedlicher Hämatokrit-Werte und mit einer wässrigen Glucoselösung durchgeführt, wobei die resultierenden Messwerte in Figur 3 mit unterschiedlichen Symbolen bezeichnet sind. Darin bezeichnen #1 bis #3 die Nummern von Proben mit unterschiedlichen Hämatokritkonzentrationen (sehr hoch - Normalbereich - sehr niedrig); "ags" bedeutet "aqueous glucose solution". Man erkennt eine erstaunlich gute Korrelation zwischen der Glucosekonzentration und dem $R_{s/l}$-Wert, wobei die Messwerte weitgehend unabhängig sind von dem Hämatokrit-Wert, also von der Konzentration der Blutkörperchen in der Probe.

Figur 3 zeigt ein erstes Beispiel einer bevorzugten Ausführungsform der Erfindung, bei der zwei LRFI-Werte in Beziehung zueinander gesetzt werden. Dabei wurden $R_{s/l}$ berechnet gemäß

$$R_{s/1} = \frac{A_2 \cdot \tau_2}{A_1 \cdot \tau_1}$$

wobei A die Amplitude, also ein Maß der Intensität und $\tau$ die Fluoreszenzlebensdauer der beiden mit unterschiedlicher Lebensdauer fluoreszierenden Spezies bezeichnet. Der Wert $R_{s/l}$ wird gegen die Analytkonzentration kalibriert und dann in dem Auswertealgorithmus als Maß für die Analytkonzentration verwendet.

Es gibt zahlreiche andere Möglichkeiten, LRFI-Werte derartig zueinander in Beziehung zu setzen, dass eine Rechengröße gebildet wird, die mittels einer Kalibration als Maß für die gesuchte Analytkonzentration verwendet werden kann. Beispielsweise kann eine gemittelte Fluoreszenzlebensdauer $\tau_m$ berechnet werden gemäß

$$\tau_m = \frac{A_1\tau_1 + A_2\tau_2}{A_1 + A_2}$$

Gemäß einer besonders bevorzugten Ausführungsform erfolgt eine flächengewichtete Mittlung gemäß

$$\tau_m = \frac{A_1\tau_1^2 + A_2\tau_2^2}{A_1\tau_1 + A_2\tau_2}$$

Allen diesen Beispielen ist gemeinsam, dass die Fluoreszenzlebensdauern und die Fluoreszenzintensitäten zweier fluoreszierender Spezies als für die Analyse charakteristische Messgrößen verwendet werden. Sie werden jeweils gemäß einer vordefinierten mathematischen Gleichung in Beziehung zueinander gesetzt, um eine Rechengröße zu ermitteln, die gegen die gesuchte Analytkonzentration kalibriert werden kann. Die Verwendung von zwei simultan gemessenen Messwerten und die Bildung eines Quotienten führt dazu, dass Messfehler, die beide Fluoreszenzmesswerte in gleicher Weise beeinflussen, beispielsweise durch Verunreinigungen oder kleine Beschädigungen im optischen Messkanal, nicht oder nur in sehr viel geringerem Umfang das Messergebnis negativ beeinflussen, als bei vorbekannten Messungen der Fluoreszenzintensität.

Die Messung von LRFI-Werten ist ein Beispiel dafür, dass im Rahmen der Erfindung nicht notwendigerweise eine Messung einer Fluoreszenzlebensdauer (im strengen Sinne) notwendig ist. Wie oben dargelegt, kann die Messeinrichtung des Auswertegerätes auch so ausgebildet sein, dass sie eine Fluoreszenzintensität eines definiert gesetzten Zeitfensters erfasst und damit ein LRFI-Wert gemessen wird, wobei vorzugsweise zwei oder mehrere derartige LRFI-Werte, ähnlich wie in den vorstehenden Beispielen erläutert, in Beziehung zueinander gesetzt werden, um eine Rechengröße zu ermitteln, die gegen die Analytkonzentration geeicht werden kann.

Figur 4 zeigt die Korrelation von erfindungsgemäß ermittelten Glucosekonzentrationswerten $C_m$ mit Glucosewerten $C_{ref}$, die mit einer Referenzmethode ermittelt wurden. Auch hier zeigt sich, dass die auf der Grundlage der Fluoreszenzlebensdauer ermittelten Analysewerte erstaunlich gut mit den Werten der Referenzmethode übereinstimmen.

Die Erfindung ist nicht nur für das beschriebene System anwendbar, bei dem die Glucose mit Hilfe von GlucDH und NAD bestimmt wird. Vielmehr lässt sie sich ganz allgemein für Fälle anwenden, bei denen ein Fluorophor verwendet wird, der in mindestens zwei Spezies mit unterschiedlichen Fluoreszenziebensdauern fluoresziert. Insbesondere eignet sich die Erfindung für Tests, bei denen die Reaktion der Probe mit dem Reagenzsystem eine Komplexbildung unter Beteiligung von mindestens zwei Molekülen einschließt, wobei der Fluorophor eines der mindestens zwei Moleküle ist. Die an der Komplexbildung beteiligten Moleküle sind vorzugsweise ein Enzym/Coenzym-Paar, wobei das Coenzym vorzugsweise der Fluorophor ist.

Bevorzugt ist der Fluorphor ein Coenzym, ausgewählt aus der Gruppe bestehend aus NADH/H$^+$ und NADPH/H$^+$.

Vorzugsweise ist das Enzym eines im Rahmen der Erfindung verwendeten Enzym/Coenzym-Paares ausgewählt aus der Gruppe bestehend aus Glucose-Dehydrogenase (E.C.1.1.1.47), Lactat-Dehydrogenase (E.C.1.1.1.27, 1.1.1.28), Malat-Dehydrogenase (E.C. 1.1.1.37), Glycerin-Dehydrogenase (E.C.1.1.1.6), Alkohol-Dehydrogenase (E.C.1.1.1.1) oder Aminosäure-Dehydrogenase, z.B. L-Aminosäure-Dehydrogenase (E.C. 1.4.1.5).

Das Reagenzsystem kann in Form eines "Analysekit" verwendet werden, wobei die Analysekit die notwendigen Reagenzien und Hilfsmittel in einem oder mehreren Behältnissen oder Trägern enthält. Vorzugsweise ist der Analysekit als Analyseelement ausgebildet, wobei das Analyseelement die Reagenzien des Reagenzsystems in trockener Form enthält.

Wie beschrieben korreliert die Fluoreszenzlebensdauer so gut mit der Konzentration des Analyten, dass sie selbständig als für die Konzentration charakteristische Messgröße in dem Auswertealgorithmus für die Berechnung der Analytkonzentration verwendet werden kann. Vorzugsweise wird sie jedoch in Kombination mit einer anderen die Konzentration charakterisierenden Messgröße, insbesondere der Fluoreszenzintensität, aber auch der optischen Absorption verwendet. Dabei kann sie auch zur Anwendung kommen, um Messfehler zu kompensieren, die durch Störgrößen, insbesondere die Temperatur der Probe oder deren Hämatokrit, verursacht werden.

**Patentansprüche**

1. Verfahren zur Bestimmung der Konzentration eines Analyten in einer flüssigen Probe (6) mittels eines Reagenzsystems, wobei

die Reaktion der Probe (6) mit dem Reagenzsystem zu einer Änderung der Menge eines Fluorophors führt,

die Menge des Fluorophors mit der Konzentration des Analyten korreliert,

eine für die Menge des Fluorophors charakteristische Messgröße gemessen und

die Konzentration des Analyten mittels eines Auswertealgorithmus auf Basis der Messgröße ermittelt wird,

**dadurch gekennzeichnet, dass** die Fluoreszenzlebensdauer des Fluorophors bei der Ermittlung der Konzentration des Analyten verwendet wird.

2. Verfahren nach Anspruch 1, **dadurch gekennzeichnet, dass** die Reaktion der Probe mit dem Reagenzsystem eine Komplexbildung unter Beteiligung von mindestens zwei Molekülen einschließt, wobei der Fluorophor eines der mindestens zwei Moleküle ist.

3. Verfahren nach Anspruch 2, **dadurch gekennzeichnet, dass** die mindestens zwei an der Komplexbildung beteiligten Moleküle ein Enzym/Coenzym-Paar einschließen.

4. Verfahren nach Anspruch 3, **dadurch gekennzeichnet, dass** das Coenzym der Fluorophor ist.

5. Verfahren nach einem der vorhergehenden Ansprüche, **dadurch gekennzeichnet, dass** der Fluorophor ausgewählt ist aus der Gruppe bestehend aus NADH/H$^+$ und NADPH/H$^+$.

6. Verfahren nach Anspruch 3, **dadurch gekennzeichnet, dass** das Enzym ausgewählt ist aus der Gruppe bestehend aus Glucose-Dehydrogenase (E.C.1.1.1.47), Lactat-Dehydrogenase (E.C. 1.1.1.27, 1.1.1.28), Malat-Dehydrogenase (E.C. 1.1.1.37), Glycerin-Dehydrogenase (E.C.1.1.1.6), Alkohol-Dehydrogenase (E.C.1.1.1.1) oder Aminosäure-Dehydrogenase, z.B. L-Aminosäure-Dehydrogenase (E.C.1.4.1.5).

7. Verfahren nach einem der vorhergehenden Ansprüche, **dadurch gekennzeichnet, dass** der Analyt Glucose ist.

8. Verfahren nach einem der vorhergehenden Ansprüche, **dadurch gekennzeichnet, dass** die gemessene Fluoreszenzlebensdauer des Fluorophors in dem Auswertealgorithmus als eine für die Konzentration charakteristische Messgröße verwendet wird.

9. Verfahren nach einem der vorhergehenden Ansprüche, **dadurch gekennzeichnet, dass** die gemessene Fluoreszenzlebensdauer in dem Auswertealgorithmus in Kombination mit einer anderen für die Konzentration charakteristischen Messgröße, insbesondere der Fluoreszenzintensität, verwendet wird.

10. Verfahren nach Anspruch 9, **dadurch gekennzeichnet, dass** die gemessene Fluoreszenzlebensdauer in dem Auswertealgorithmus zur Kompensation von Messfehlern verwendet wird, die durch Störgrößen verursacht werden.

11. Verfahren nach einem der vorhergehenden Ansprüche, **dadurch gekennzeichnet, dass** die Messung der Fluoreszenzlebensdauer verwendet wird, um einen Lebensdauer-bezogenen Fluoreszenzintensitätswert zu bestimmen.

12. Verfahren nach Anspruch 11, **dadurch gekennzeichnet, dass** die Fluoreszenzintensität für mindestens zwei verschiedene Fluoreszenziebensdauern gemessen wird, um jeweils Lebensdauer-bezogene Fluoreszenzintensitätswerte zu bestimmen.

13. Verfahren nach Anspruch 12, **dadurch gekennzeichnet, dass** die beiden Lebensdauer-bezogenen Fluoreszenzintensitätswerte in Beziehung zueinander gesetzt werden.

14. Verfahren nach einem der vorhergehenden Ansprüche, **dadurch gekennzeichnet, dass** das Reagenzsystem in einem Analyseelement integriert ist.

15. Analysesystem zur Bestimmung der Konzentration eines Analyten in einer flüssigen Probe mittels eines Verfahrens nach einem der vorhergehenden Ansprüche,
mit
einem Analysekit, enthaltend ein Reagenzsystem, dessen Reaktion mit der Probe zu einer Änderung der Menge eines Fluorophors führt, wobei die Menge des Fluorophors mit der Konzentration des Analyten korreliert, und
einem Auswertegerät (3), welches eine Messeinheit zur Messung einer für die Menge des Fluorophors charakteristischen Messgröße einschließt und welches eine Auswerteeinheit einschließt, um mittels eines Auswertealgorithmus auf der Basis der Messgröße die Konzentration des Analyten zu ermitteln, **dadurch gekennzeichnet, dass** die Messeinheit (8) des Auswertegerätes dazu ausgebildet ist, die Fluoreszenzlebensdauer des Fluorophors zu messen und/oder zu berücksichtigen.

16. Analysesystem nach einem der vorhergehenden Ansprüche, **dadurch gekennzeichnet, dass** der Analysekit als Analyseelement (2) ausgebildet ist, das Reagenzien des Reagenzsystems in trockener Form enthält.

**Fig. 1**

**Fig. 2**

Fig. 3

Fig. 4

**Europäisches Patentamt**

# EUROPÄISCHER RECHERCHENBERICHT

Nummer der Anmeldung

EP 07 01 5084

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EVANS N D ET AL: "Glucose-dependent changes in NAD(P)H-related fluorescence lifetime of adipocytes and fibroblasts in vitro: Potential for non-invasive glucose sensing in diabetes mellitus" 1. August 2005 (2005-08-01), JOURNAL OF PHOTOCHEMISTRY AND PHOTOBIOLOGY B: BIOLOGY, ELSEVIER SCIENCE S.A., BASEL, CH, PAGE(S) 122-129 , XP004984395 ISSN: 1011-1344 * Seite 123, linke Spalte, Absatz 2 - Seite 124, rechte Spalte, Absatz 2 * * Seite 125, rechte Spalte, Absatz 2 * * Seite 128, linke Spalte, Absatz 4 * * Abbildung 2 * | 1-15 | INV. C12Q1/32 C12Q1/54 |
| Y | * Seite 123, rechte Spalte, Absatz 3 - Seite 124, rechte Spalte, Absatz 2 * | 16 | |
| X | WO 2004/044557 A (ARGOSE INC [US]; WORKMAN JEROME J [US]; LAMBERT CHRISTOPHER R [US]; CO) 27. Mai 2004 (2004-05-27) * Seite 63, Zeile 13 - Zeile 23 * * Seite 65, Zeile 4 - Zeile 15 * * Seite 102, Zeile 24 - Seite 103, Zeile 9 * * Seite 104, Zeile 23 - Seite 111, Zeile 5 * * Beispiel 14 * * Abbildung 16 * | 1-14 | |
| Y | WO 03/097859 A (HOFFMANN LA ROCHE [CH]; ROCHE DIAGNOSTICS GMBH [DE]; HORN CARINA [DE];) 27. November 2003 (2003-11-27) * Beispiele 2,3 * | 16 | RECHERCHIERTE SACHGEBIETE (IPC) C12Q |
| D,A | US 5 485 530 A (LAKOWICZ JOSEPH R [US] ET AL) 16. Januar 1996 (1996-01-16) * das ganze Dokument * | 1-16 | |

Der vorliegende Recherchenbericht wurde für alle Patentansprüche erstellt

| Recherchenort | Abschlußdatum der Recherche | Prüfer |
|---|---|---|
| Den Haag | 19. Oktober 2007 | Graf, Eva |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 07 01 5084

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-10-2007

| Im Recherchenbericht angeführtes Patentdokument | Datum der Veröffentlichung | Mitglied(er) der Patentfamilie | | Datum der Veröffentlichung |
|---|---|---|---|---|
| WO 2004044557 A | 27-05-2004 | AU | 2003287735 A1 | 03-06-2004 |
| | | US | 2007020181 A1 | 25-01-2007 |
| WO 03097859 A | 27-11-2003 | AT | 345396 T | 15-12-2006 |
| | | AU | 2003232790 A1 | 02-12-2003 |
| | | AU | 2003240260 A1 | 02-12-2003 |
| | | AU | 2003240666 A1 | 02-12-2003 |
| | | BR | 0309947 A | 01-03-2005 |
| | | BR | 0311175 A | 15-03-2005 |
| | | CA | 2486950 A1 | 27-11-2003 |
| | | CA | 2493918 A1 | 27-11-2003 |
| | | CN | 1653190 A | 10-08-2005 |
| | | CN | 1653189 A | 10-08-2005 |
| | | DK | 1504113 T3 | 05-03-2007 |
| | | WO | 03097863 A1 | 27-11-2003 |
| | | WO | 03097864 A1 | 27-11-2003 |
| | | EP | 1504113 A2 | 09-02-2005 |
| | | EP | 1504115 A1 | 09-02-2005 |
| | | EP | 1504116 A1 | 09-02-2005 |
| | | ES | 2275095 T3 | 01-06-2007 |
| | | JP | 2005528896 T | 29-09-2005 |
| | | JP | 2005528897 T | 29-09-2005 |
| | | JP | 2005532796 T | 04-11-2005 |
| | | MX | PA04011103 A | 14-02-2005 |
| | | MX | PA04011220 A | 14-02-2005 |
| | | US | 2005214891 A1 | 29-09-2005 |
| | | US | 2006099327 A1 | 11-05-2006 |
| US 5485530 A | 16-01-1996 | EP | 0568596 A1 | 10-11-1993 |
| | | WO | 9213265 A1 | 06-08-1992 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0293732 A2
- US 20050214891 A1
- US 20060003397 A1
- US 5485530 B
- EP 0561653 A1
- US 3802842 A
- US 4061468 A
- US 20060003397 A

**In der Beschreibung aufgeführte Nicht-Patentliteratur**

- **J.R. LAKOVICZ.** Principles of Fluorescence Spectroscopy. Springer Science and Business Media, 2006
- **T. G. SCOTT et al.** Emission Properties of NADH. Studies of Fluorescence Lifetimes ... *J. Am. Chem. Soc.,* 1970, 687-695